Europäisches Patentamt

European Patent Office

Office européen des brevets

(11) Publication number: **0 081 491**
**B1**

# EUROPEAN PATENT SPECIFICATION

(45) Date of publication of patent specification: 04.11.87

(51) Int. Cl.⁴: **A 61 F 13/20**

(21) Application number: 81902094.2

(22) Date of filing: 22.06.81

(86) International application number:
PCT/US81/00873

(87) International publication number:
WO 83/00012 06.01.83 Gazette 83/01

(54) APPLICATOR AND TAMPON.

(43) Date of publication of application:
22.06.83 Bulletin 83/25

(45) Publication of the grant of the patent:
04.11.87 Bulletin 87/45

(84) Designated Contracting States:
AT CH DE FR GB LI LU NL SE

(56) References cited:
US-A-2 922 423
US-A-3 058 469
US-A-3 499 447
US-A-3 749 093
US-A-4 211 225

(73) Proprietor: CUNNINGHAM, Thomas W.
3580 Emerywood Lane
Orlando, FL 32806 (US)

(72) Inventor: CUNNINGHAM, Thomas W.
3580 Emerywood Lane
Orlando, FL 32806 (US)

(74) Representative: Lambert, Hugh Richmond et al
D. YOUNG & CO. 10 Staple Inn
London, WC1V 7RD (GB)

## Description

The present invention relates to applicators for inserting a tampon of other insertion member into an orifice.

During the menstruation cycle, women customarily insert an oblong porous object customarily referred to as a tampon into the vagina to absorb discharge fluids from the body. During the insertion process, the body muscles tend to contract, thus making entry of the tampon uncomfortable.

There have been numerous tampon wrappings and applicators devised in the prior art to attempt to avoid this discomfort.

In US—A—3,749,093, there is disclosed an insertable device package for tampons, in which the insertion device comprises an elongated sheath having a pair of side walls separated by a convolution at the rear end of the sheath. The tampon is inserted by axially pushing the inwardly projecting rear end of the sheath thereby to force the tampon out of the other end and causing the convolution to roll inwardly and extending the inner sidewall during the insertion process.

In US—A—2,922,422, there is disclosed a cellulose outer shell as an applicator for a tampon. Similar arrangements are disclosed in US—A—2,922,423 and US—A—3,499,447 but have little relevance to the present invention.

Other devices are shown in US—A—3,058,469, US—A—3,135,262, US—A—3,358,636, US—A—3,068,867, US—A—4,048,998, US—A—2,413,480, US—A—2,105,710, US—A—2,401,585 and DE—A—2,406,823.

In particular contrast to US—A—3,749,093 the present invention contemplates an improved applicator for an insertion member such as a tampon. Essentially this is achieved by a device similar to that in US—A—3,749,093, but in which the convolution is at the forward end, the tampon being inserted effectively by peeling the sheath backward, rather than pushing the tampon forward.

In one aspect the invention provides an applicator in combination with a tampon or other insertion member for insertion thereof into an orifice comprising a flexible tubular sleeve positioned about the member to be inserted and doubled upon itself to form an inner wall or layer and an outer layer superimposed on top of the inner layer, said layers being joined by a convolution at one end of the applicator, said applicator being insertable into said orifice and operable to deposit the member therein by moving the outer and inner layers of the tubular sleeve relatively to each other with concomitant rolling of said sleeve at convolution thereby to remove the sleeve and leave said member *in situ* in said orifice, wherein said convolution is at the forward end of the applicator, the insertion member thus being in surface contact with the inner of the two layers of the sleeve, and whereby the member may be deposited in said orifice, after insertion of said applicator, by grasping the outer layers of said sleeve at its open rear end, and drawing that outer layer relative to the inner layer in a rearward direction, thereby causing the sleeve to unroll about the convolution at the forward end of the applicator and deposit the member in the orifice.

In a second aspect the invention provides an applicator in combination with a tampon or other insertion member for insertion thereof into an orifice comprising a flexible tubular sleeve having an open end and being positioned about the member to be inserted and which, after insertion of the applicator into said orifice, is removable to leave the member *in situ* in said orifice, wherein the end extends forwardly beyond the insertion member and wherein the flexible tubular sleeve is provided with a longitudinal slit extending partially along the length of the sleeve from said forward end and which enables said sleeve, at its forward end, to be folded back on itself, to form a folded sleeve with a convolution at its forward end, and from there to be removed entirely by grasping the rearwardly directed open end of the outer layer of the now folded sleeve and drawings that outer layer relative to the inner layer in a rearward direction, thereby causing the sleeve to unroll about the convolution and deposit the member in the orifice.

A preferred embodiment of the present invention contemplates an applicator and tampon, in which the tampon is formed of an oblong absorbent member having a central axis and a forward end adapted to be extended first into the vagina. A flexible applicator sleeve is fitted about the tampon and overlapping a portion of the forward end of the tampon, with a convolution along the sleeve permitting the sleeve to be rolled along a direction substantially parallel with the central axis during or after movement of the tampon into the vagina. For these purposes, the term "convolution" means a single roll, with a portion of the sleeve about another portion of the sleeve.

In one embodiment of the present invention, the flexible applicator sleeve surrounds the tampon and has a longitudinal slit. The slit extends from one end of the tampon to one extremity of the sleeve, and may preferably have perforations along the line which is aligned with the slit, but adjacent the tampon. The sleeve may be fitted with a second slit extending from the other extremity of the sleeve, the second slit being out of alignment with the first slit. In use, the first slit permits the sleeve to be rolled backward substantially parallel with the central axis of the tampon to form the convolution, and thereafter facilitate relative movement of the tampon with respect to the sleeve. In this regard, the conventional stick or similar means may be used to push the tampon upward, opens the distal end of the sleeve to permit access to the tampon.

The invention is further described with reference to the accompanying drawings, in which:

Figures 1, 2 and 3 illustrate one embodiment of the present invention and the consecutive steps in the use of that embodiment;

Figures 4, 5 and 6 illustrate consecutive steps in the use of another embodiment of the applicator and tampon of the present invention.

Referring now to the drawings, the applicator and tampon, referred to generally by the reference numeral (10), includes a tampon (12) comprising an absorbent fiber body, which is compressed to such an extent that it is substantially stable under normal atmospheric conditions but expands when wetted as during its intended use. The tampon (12) is provided with a conventional insertion rod (14) which may comprise wood, paper or any other material of a suitable nature, and which is appropriately joined at the distal end (16) of the tampon (12). The applicator and tampon is further provided with a conventional withdrawal string (18) suitably anchored to the tampon (12) and exits adjacent to the distal end (16).

The applicator, referred to generally by the reference numeral (20), comprises a flexible sleeve about the tampon and overlapping a portion of the forward end (22) of the tampon (12). A suitable material for use as the sleeve (20) may be, for example, polyethylene or polypropylene or other material having sufficient flexibility to form the desired convolution.

The sleeve (20) is wrapped about the tampon (12) in such a manner as to form a convolution (24) toward the forward end (22) of the tampon (12), the convolution forming an opening (26) axial with the central axis of the tampon (12) and overlapping the forward end (22). The convolution (24) thus separates the sleeve (20) into sidewalls (28) and (30), the first sidewall (28) being next adjacent to the tampon (12), and the outer sidewall (30) being next adjacent to the first wall (28).

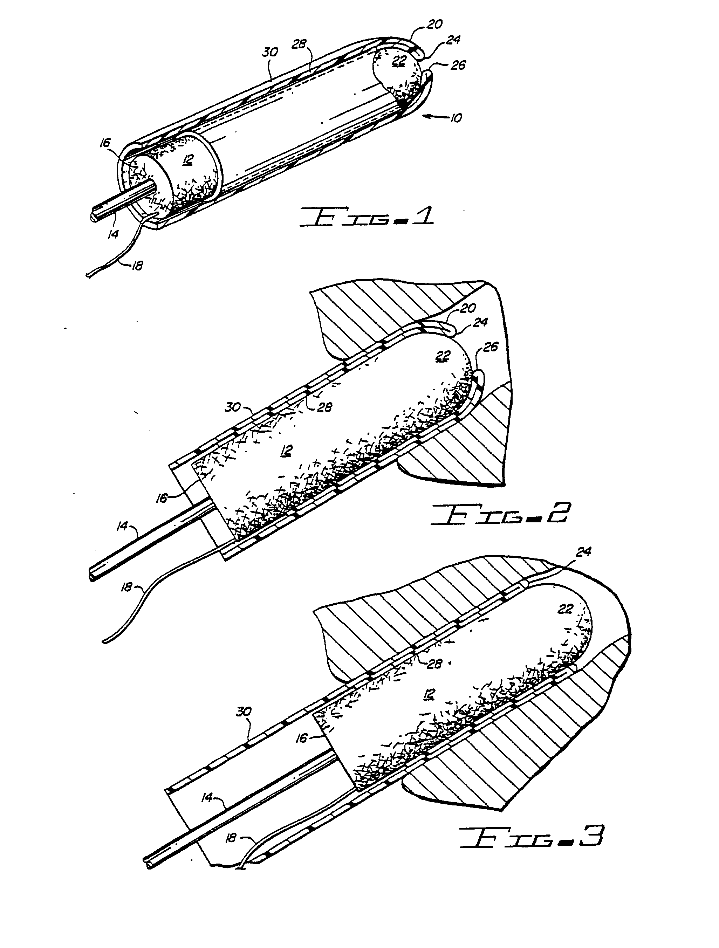

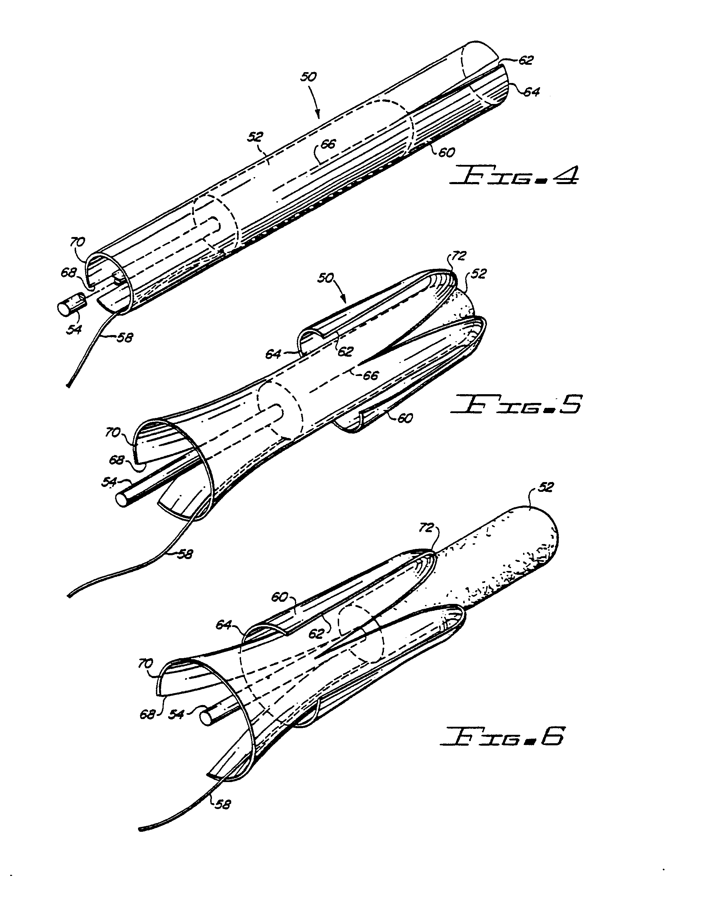

The manner in which the applicator and tampon (10) is employed will now be described with reference to Figures 2 and 3.

Noting Figure 2, the applicator and tampon (10) are inserted into the vagina, with the muscle tissue coming in contact with the outer wall (30) of the sleeve (20). As the tampon (12) is forced into the vagina by application of pressure on the insertion rod (14), the sleeve (20) rolls at the convolution (24), with the outer wall (30) remaining substantially stationary. After the tampon is inserted in the desired manner, the insertion rod (14) may be removed in a conventional fashion by rotation thereof, and the sleeve (20) may be removed by simply pulling the outer extremity of the outside wall (30) away from the vagina. Alternatively, the tampon (12) is placed into position and the sleeve (20) is removed while the tampon remains relatively stationary. It will thus be seen that a tampon and applicator constructed in accordance with the present invention reduces significantly the amount of tampon insertion discomfort. Further, the use of the thin poly-resin sleeve (20) provides relatively easy entry during the insertion process.

It will be understood that the sleeve (20) may be coated with an appropriate lubricant. It will be further understood that the sleeve (20) may be adapted for use with tampons of different sizes and configurations, i.e., rectangular or other shapes.

While the applicator has been described above for use with tampons, it will be understood that the applicator may be employed with other products as well.

A second embodiment of the applicator and tampon in accordance with the present invention will now be described with reference to Figures 4, 5, and 6. The combination applicator and tampon, referred to generally by the reference numeral (50), includes a tampon (52) having a longitudinal central axis. The tampon (52) includes a conventional insertion rod (54) and a withdrawal string (58) suitably anchored to the tampon (52) adjacent the distal end thereof. The applicator, referred to generally by the reference numeral (60), comprises a flexible sleeve about the tampon (52) and overlapping the forward and rearward ends of the tampon.

As is clearly shown in Figure 4, the applicator sleeve (60) surrounds the tampon (52) and has a longitudinal slit (62) along a portion thereof substantially parallel with the central axis of the tampon. This slit (62) extends from one end of the tampon (52) through the forward extremity (64) of the sleeve (60). With continuous reference to Figure 4, the sleeve (60) further includes a perforated line (66) which is aligned with the slit (62) adjacent the tampon (52). The applicator sleeve (60) of Figures 4, 5, and 6 further preferably include a second slit (68) in the sleeve extending from the other extremity (70), the slit being out of alignment with the first slit (62). In the embodiment shown in Figure 4, second slit (68) is out of alignment with slit (62) by 180°. However, it will be understood that the second slit (68) need not necessarily be parallel with the central axis of the tampon (52), but preferably is so parallel with that axis.

The mode of operation of the applicator and tampon (50) of Figures 4, 5, and 6 will now be described with reference to Figures 5 and 6.

First, note in Figure 5, the applicator sleeve (60) is initially folded back along the convolution (72) adjacent the forward extremity of the tampon (52), and the tampon and applicator combination (50) has been inserted. As is shown in Figure 6, the insertion rod (54) is then pushed forward, causing the tampon (52) to move forward into the appropriate position in the vagina. The use of the insertion rod (54) (or other means, such as the user's finger) is facilitated by the wider opening at the distal extremity (70) of the sleeve (60), effectuated by the second slit (68). The sleeve (60) may then be removed along with the insertion rod (54).

While the second embodiment of Figures 4, 5, and 6 has been disclosed as including both first and second slits (62) and (68), as well as a perforated line (66), it will be appreciated by those skilled in the art that the arrangement here disclosed may include only the first or second slit alone or in combination with the perforated line (66).

## Claims

1. An applicator in combination with a tampon or other insertion member for insertion thereof into an orifice, comprising a flexible tubular sleeve (20) positioned about the member (12) to be inserted and doubled upon itself to form an inner wall or layer (28) and an outer layer (30) superimposed on top of the inner layer (28), said layers being joined by a convolution (24) at one end of the applicator, said applicator being insertable into said orifice and operable to deposit the member (12) therein by moving the outer and inner layers (28, 30) of the tubular sleeve relatively to each other with concomitant rolling of said sleeve at convolution (24) thereby to remove the sleeve (20) and leave said member *in situ* in said orifice, characterised in that said convolution (24) is at the forward end of the applicator, and that the insertion member (12) is in surface contact with the inner (28) of the two layers (28, 30) of the sleeve (20), whereby the member (12) may be deposited in said orifice, after insertion of said applicator, by grasping the outer layer (30) of said sleeve (20) at its open rear end, and drawing that outer layer relative to the inner layer in a rearward direction, thereby causing the sleeve to unroll about the convolution (24) at the forward end of the applicator and deposit the member in the orifice.

2. A combination according to claim 1, including a rigid rod (14) detachably secured to the rear end (16) of the member (12) to be inserted and extending axially through the open rear end of the applicator and which serves (a) to assist insertion of the applicator into the orifice and (b) to hold the member (12) in place in the orifice as the sleeve (20) is removed.

3. An applicator in combination with a tampon or other insertion member for insertion thereof into an orifice, comprising a flexible tubular sleeve (60) having an open end (64) and being positioned about the member (52) to be inserted and which, after insertion of the applicator into said orifice, is removable to leave the member (52) *in situ* in said orifice, characterised in that the open end (64) extends forwardly beyond the insertion member (52) and that the flexible tubular sleeve (60) is provided with a longitudinal slit (62) extending partially along the length of the sleeve from said forward end (64) and which enables said sleeve (60), at its forward end, to be folded back on itself, to form a folded sleeve with a convolution (72) at its forward end, and from there to be removed entirely by grasping the rearwardly directed open end (64) of the outer layer of the now folded sleeve (60) and drawing that outer layer relative to the inner layer in a rearward direction, thereby causing the sleeve (60) to unroll about the convolution (72) and deposit the member (52) in the orifice.

4. A combination according to claim 3, characterised in that the tubular sleeve (60) is further provided with a line of perforations (66) extending longitudinally of the sleeve from the inner end of said slit (62).

5. A combination according to claim 3 or 4, characterised in that the tubular sleeve (60) is further provided with a second longitudinal slit (68) extending partially along the length of the sleeve from the opposite end (70), said second slit being out of axial alignment with said first slit (62).

6. A combination according to claim 3, 4 or 5, including a rigid rod (54) detachably secured to the rear end of the member (52) to be inserted and extending through the open rear end of the applicator and which serves (a) to assist the insertion of the applicator into the orifice and (b) to hold the member (52) in place as the sleeve (60) is removed.

## Patentansprüche

1. Applikator in Kombination mit einem Tampon oder einem anderen Einführteil zum Einführen desselben in eine Öffnung mit einer flexiblen röhrenförmigen Hülse (20), die um das einzuführende Teil (12) angeordnet und auf sich selbst umgebogen ist, im eine Innenwand oder -schicht (28) und eine Außenschicht (30), die über der Innenschicht (28) gelagert ist, zu bilden, wobei diese Schichten durch eine Faltung (24) am einen Ende des Applikators miteinander verbunden sind, der Applikator in die Öffnung einführbar und darin betätigbar ist, um das Teil (12) darin abzulagern, indem man die Außen- und Innenschicht (28, 30) der röhrenförmigen Hülse in Relation zueinander mit begleitendem Abrollen der Hülse an der Faltung (24) bewegt und so die Hülse (20) entfernt und das Teil in situ in der Öffnung zurückläßt, dadurch gekennzeichnet, daß die Faltung (24) sich am Vorderende des Applikators befindet und daß das Einführteil (12) in Oberflächenberührung mit der inneren (28) der beiden Schichten (28, 30) der Hülse (20) steht, wodurch das Teil (12) nach dem Einführen des Applikators in der Öffnung abgelagert werden kann, indem man die Außenschicht (30) der Hülse (20) an ihrem offenen Hinterende ergreift und jene Außenschicht in Bezug auf die Innenschicht nach hinten zieht und dabei bewirkt, daß die Hülse sich um die Faltung (24) am Vorderende des Applikators abwickelt und das Teil in der Öffnung abgelagert wird.

2. Kombination nach Anspruch 1 mit einem starren Stab (14), der an dem Hinterende (16) des Teils (12) lösbar befestigt ist, um eingeführt zu werden und sich durch das offene Hinterende des Applikators axial zu erstrecken, und das dazu dient, (a) die Einführung des Applikators in die Öffnung zu unterstützen und (b) das Teil (12) an seiner Stelle an der Öffnung zu halten, wenn die Hülse (20) entfernt wird.

3. Applikator in Kombination mit einem Tampon oder einem anderen Einführteil zum Einführen desselben in eine Öffnung mit einer flexiblen röhrenförmigen Hülse (60), die ein offenes Ende (64) hat und um das einzuführende Teil (52) herum angeordnet ist und die nach dem Einführen des Applikators in die Öffnung entfernbar ist, um das Teil (52) in situ in der Öffnung zurückzulassen, dadurch gekennzeichnet, daß das offene Ende (64) sich nach vorn über das Einführteil (52) hinaus erstreckt und daß die flexible röhrenförmige Hülse (60) mit einem Längsschlitz (62) versehen ist, der sich teilweise entlang der Länge der Hülse von dem Vorderende (64) aus erstreckt und der es ermöglicht, die Hülse (60) an ihrem Vorderende auf sich selbst zurückzufalten und so eine gefaltete Hülse mit einer Faltung (72) an ihrem Vorderende zu bilden, und von dort vollständig zu entfernen, indem man das nach hinten gerichtete offene Ende (64) der Außenschicht der nun gefalteten Hülse (60) ergreift und jene Außenschicht bezüglich der Innenschicht nach hinten zieht und so bewirkt, daß die Hülse (60) um die Faltung (72) abgewickelt und das Teil (52) in der Öffnung abgelagert wird.

4. Kombination nach Anspruch 3, dadurch gekennzeichnet, daß die röhrenförmige Hülse (60) weiterhin mit einer Perforationslinie (66) versehen ist, die sich längs der Hülse von dem Innenende des Schlitzes (62) aus erstreckt.

5. Kombination nach Anspruch 3 oder 4, dadurch gekennzeichnet, daß die röhrenförmige Hülse (60) außerdem mit einem zweiten Längsschlitz (68) versehen ist, der sich teilweise entlang der Länge der Hülse von dem entgegengesetzten Ende (70) aus erstreckt, wobei dieser zweite Schlitz axial nicht mit dem ersten Schlitz (62) fluchtet.

6. Kombination nach Anspruch 3, 4 oder 5 mit einem starren Stab (54), der lösbar an dem Hinterende des Teils (52) befestigt ist, um durch das offene Ende des Applikators eingeführt zu werden und sich zu erstrecken und der dazu dient, (a) das Einführen des Applikators in die Öffnung zu unterstützen und (b) das Teil (52) an seiner Stelle zu halten, wenn die Hülse (60) entfernt wird.

**Revendications**

1. Applicateur en combinaison avec un tampon ou autre élément susceptible d'être introduit, pour l'introduction de celui-ci dans un orifice, comprenant un manchon tubulaire flexible (20) positionné autour de l'élément (12) à introduire et replié sur lui-même pour former un paroi ou couche interne (28) et une couche externe (30) superposée au sommet de la couche interne (28), lesdites couches étant raccordées par une circonvolution (24) à une extrémité de l'applicateur, ledit applicateur étant susceptible d'être introduit dans ledit orifice et d'être mis en oeuvre pour y déposer l'élément (12) en déplaçant les couches externe et interne (28, 30) du manchon tubulaire l'une par rapport à l'autre tout en faisant rouler ledit manchon à ladite circonvolution (24) de façon à retirer ledit manchon (20) et laisser ledit élément *in situ* dans ledit orifice, caractérisé en ce que ladite circonvolution (24) est à l'extrémité avant de l'applicateur et que l'élément (12) est en contact superficiel avec la couche interne (28) du manchon (20), de sorte que l'élément (12) puisse être déposé dans ledit orifice après introduction dudit applicateur, en saisissant la couche externe (30) dudit manchon (20) à son extrémité arrière ouverte et en tirant en arrière cette couche externe par rapport à la couche interne, forçant ainsi le manchon à se dérouler autour de la circonvolution (24) à l'extrémité avant de l'applicateur et à déposer l'élément dans l'orifice.

2. Combinaison selon la revendication 1 comportant une tige rigide (14) fixée de façon détachable à l'extrémité arrière (16) de l'élément (12) à introduire et s'étendant axialement à travers l'extrémité arrière ouverte de l'applicateur et qui sert (a) à aider l'introduction de l'applicateur dans l'orifice, et (b) à maintenir l'élément (12) en place dans l'orifice tandis que le manchon (20) est dégagé.

3. Applicateur en combinaison avec un tampon ou autre élément susceptible d'être introduit, pour l'introduction de celui-ci dans un orifice, comprenant un manchon tubulaire flexible (60) ayant une extrémité ouverte (64) et positionné autour de l'élément (52) à introduire et qui, après introduction de l'applicateur dans ledit orifice, peut être retiré pour laisser l'élément (52) *in situ* dans ledit orifice, caractérisé en ce que l'extrémité ouverte (64) s'étend en avant et au-delà de l'élément (52) et en ce que ledit manchon tubulaire flexible (60) est pourvu d'une première fente longitudinale (62) s'étendant partiellement le long de la longueur du manchon à partir de ladite extrémité avant (64) et qui permet audit manchon (60), à son extrémité avant, d'être rabattu sur lui-même pour former un manchon plié avec une circonvolution (72) à son extrémité avant, et à partir de là, d'être entièrement retiré en saisissant l'extrémité ouverte (64) dirigée vers l'arrière, de la couche externe du manchon (60) ainsi plié et en tirant cette couche externe par rapport à la couche interne, vers l'arrière, forçant ainsi ledit manchon (60) à se dérouler autour de ladite circonvolution (72) et à laisser l'élément (52) dans l'orifice.

4. Combination selon la revendication 3, caractérisée en ce que le manchon tubulaire (60) est en outre pourvu d'une ligne de perforation (66) s'étendant longitudinalement sur ledit manchon à partir de l'extrémité interne de ladite fente (62).

5. Combinaison selon la revendication 3 ou 4, caractérisée en ce que le manchon tubulaire (60) est en outre pourvu d'une seconde fente longitudinale (68) s'étendant partiellement le long de la longueur du manchon à partir de l'extrémité opposée (70), ladite seconde fente n'étant par alignée axialement avec ladite première fente (62).

6. Combinaison selon la revendication 3, 4 ou 5 comportant une tige rigide (54) fixée de façon détachable à l'extrémité arrière de l'élément (52) à introduire et s'étendant à travers l'extrémité arrière ouverte de l'applicateur et qui sert (a) à aider l'introduction de l'applicateur dans l'orifice, et (b) à maintenir l'élément (52) en place tandis que le manchon (60) est dégagé.

**FIG-1**

**FIG-2**

**FIG-3**

FIG-4

FIG-5

FIG-6